# EUROPEAN PATENT SPECIFICATION

(11) **EP 2 373 787 B1**
(45) Date of publication and mention of the grant of the patent: **28.10.2015**
(21) Application number: 09795798.9
(22) Date of filing: 22.12.2009
(51) Int. Cl.: C12P 21/02, C12P 7/10

(54) **PROCESS FOR PRODUCTION OF AN ENZYMATIC PREPARATION FOR HYDROLYSIS OF CELLULOSE FROM LIGNOCELLULOSIC RESIDUES**
HERSTELLUNG EINER ENZYMATISCHEN LÖSUNG FÜR DIE HYDROLYSE VON ZELLULOSE AUS LIGNOZELLULOSEHÄLTIGEN RÜCKSTÄNDEN
PROCÉDÉ DE PRODUCTION D'UNE PRÉPARATION ENZYMATIQUE POUR L'HYDROLYSE DE CELLULOSE À PARTIR DE RÉSIDUS LIGNOCELLULOSIQUES

(30) Priority: 29.12.2008 BR PI0805560
(43) Date of publication of application: 12.10.2011
(73) Proprietor: Petróleo Brasileiro S.A. Petrobras, Rio de Janeiro, RJ (BR)
(72) Inventor: DE CASTRO, Aline Machado, CEP-20720-011 Rio de Janeiro, RJ (BR); SANT'ANNA, Lídia Maria Melo, CEP-24340-240 Niterói, RJ (BR); JUNIOR, Nei Pereira, CEP-22430-190 Rio de Janeiro, RJ (BR); GOMES, Absai daConceição, CEP-21655-330 Rio de Janeiro, RJ (BR); MENEZES, Emerson Pires, CEP-21371-021 Rio de Janeiro, RJ (BR); SILVEIRA, Claudia Julia Groposo, CEP-24240-185 Santa Rosa Niterói, RJ (BR); MOYSES, Danuza Nogueira, CEP-24230-250 Niterói, RJ (BR); BANDEIRA, Luiz Fernando Martins, CEP-20270 245 Rio de Janeiro, RJ (BR); MAEDA, Roberto Nobuyuki, CEP-20270 245 Rio de Janeiro, RJ (BR)
(74) Representative: Duckworth, Timothy John
(86) International application number: PCT/GB2009/002929
(87) International publication number: WO 2010/076552

(56) References cited:
- WO-A-2007/114729
- WO-A-2008/008793
- WO-A-2008/106757
- WO-A-2009/071996
- DESHPANDE V ET AL: "SIMULTANEOUS SACCHARIFICATION AND FERMENTATION OF CELLULOSE TO ETHANOL USING PENICILLIUM-FUNICULOSUM CELLULASE AND FREE OR IMMOBILIZED SACCHAROMYCES-UVARUM CELLS" BIOTECHNOLOGY AND BIOENGINEERING, vol. 25, no. 6, 1983, pages 1679-1684, XP002570635 ISSN: 0006-3592
- BHAT K ET AL: "The endo-(1 4)-beta-D-glucanase system of Pencillium pinophilum cellulase: Isolation, purification, and characterization of five major endoglucanase components" CARBOHYDRATE RESEARCH, PERGAMON, GB, vol. 190, no. 2, 1 January 1989 (1989-01-01), pages 279-297, XP008117784 ISSN: 0008-6215
- SKOMAROVSKY A A ET AL: "New cellulases efficiently hydrolyzing lignocellulose pulp" APPLIED BIOCHEMISTRY AND MICROBIOLOGY, KLUWER ACADEMIC PUBLISHERS-PLENUM PUBLISHERS, NE, vol. 42, no. 6, 1 December 2006 (2006-12-01), pages 592-597, XP019432668 ISSN: 1608-3024
- TENGERDY R P ET AL: "Bioconversion of lignocellulose in solid substrate fermentation" BIOCHEMICAL ENGINEERING JOURNAL 200303 NL, vol. 13, no. 2-3, March 2003 (2003-03), pages 169-179, XP002570636 ISSN: 1369-703X
- SZAKACS G ET AL: "Production of cellulase and xylanase with selected filamentous fungi by solid substrate fermentation" ACS SYMPOSIUM SERIES, AMERICAN CHEMICAL SOCIETY/OXFORD UNIVERSITY PRESS, US, vol. 655, 21 November 1996 (1996-11-21), pages 175-182, XP009130220 ISSN: 0097-6156

## Description

### FIELD OF THE INVENTION

The present invention relates to the production of an enzymatic preparation based on a microbiological process, which is capable of hydrolysing the hemicellulosic and cellulosic fractions of the fibres of lignocellulosic residues obtained from forestry and agroindustrial environments. The objective of this hydrolytic process is to generate high concentrations of sugars of the glucose type and significant concentrations of xylose, which can be fermented by microorganisms with the aim of producing ethanol.

### BACKGROUND OF THE INVENTION

Brazil possesses a substantial surplus of agroindustrial and agricultural-forestry residues and is one of the world's major producers of ethanol and cellulose. Consequently, technology that makes it possible to produce ethanol from these surplus lignocellulosic residues, besides helping to solve environmental problems, will add value to these agroindustrial and agricultural-forestry surpluses, generating economic advantages for the country.

For it to be possible to exploit the polysaccharide fractions with a view to production of ethanol, it is necessary for these fractions to be hydrolysed efficiently.

Cracking of these polysaccharides is carried out by means of a pretreatment, which consists of a reaction known as acid hydrolysis, the purpose of which is hydrolysis of the hemicellulosic fraction. The solid resulting from this stage, rich in the cellulosic fraction, still needs to be treated in order to remove the soluble lignin under alkaline conditions to ensure that the cellulosic fibre is accessible to the enzymes.

The conversion of cellulose to ethanol involves two basic steps: hydrolysis of the long chains of the cellulose molecules to sugars (glucose) and fermentation of these sugars to ethanol. In nature, these processes are started by fungi and bacteria, which secrete the enzymes that are able to hydrolyse cellulose (called cellulases), and mainly by yeasts, in the case of the fermentation of sugars to alcohol.

The main difficulty that has to be overcome relates to the microorganism itself, which must be resistant to the operating conditions, mainly with respect to the concentrations of inhibitors produced in the reaction medium.

At present, one of the main bottlenecks in the second-generation biochemical production of ethanol is the production of an enzymatic preparation that is inexpensive and displays good efficiency of hydrolysis of polysaccharides such as cellulose (whose monomer is glucose) and hemicellulose (heteropolymer whose main monomer is xylose).

Production of these enzymes has been the goal of intensive research throughout the world, with the participation of large enterprises (including oil companies) and government organizations, and is fundamental to the development of "clean" technologies.

However, the high cost of these enzymes makes their wide application unviable on a commercial scale and prevents the implementation of industrial plants.

### RELATED TECHNOLOGY

The biotechnological production of ethanol has been investigated since long ago, but has undergone considerable development in recent years. The main obstacle to be overcome relates to productivity, i.e., to achieve a process that is economically viable and has good yield, using raw material that is widely available and of low total value.

Patent document GB 2253633, which corresponds to the Brazilian patent document PI 9200100-9 dated 15/01/92, describes a process for producing ethanol from biomass, in which the substrate includes a hydrolysate of cellulose, hemicellulose and starch, aiming to produce fermentable six-carbon sugars. Fermentation uses a genetically modified yeast strain (*Brettanomyces custersii* CBS 5512) that produces the enzyme β-glucosidase, which endows this yeast with the ability to ferment both glucose and cellobiose. However, we are still without a solution to the fermentation of pentoses.

Torget et al. (US 5,705,369) describe a generic process for pre-hydrolysis of lignocellulosic materials, in which various combinations of temperature ranges and reaction time are investigated, with the aim of obtaining better percentage separation of hemicellulose and lignin from cellulose.

Warzwoda et al. (BR 0600409-1) describe a process for production of cellulolytic and hemicellulolytic enzymes from residues (wood from leafy species and cereal straw). Said residues are used as the initial carbon source for obtaining these enzymes, using genetically improved, and notably recombined, strains of *Trichoderma reesei.* The wild-type strains of this microorganism have the capacity to secrete, in the presence of an inducer substrate (cellulose, for example), the enzymatic complex considered to be the most suitable for the hydrolysis of cellulose. It is therefore a process for production of cellulolytic and/or hemicellulolytic enzymes produced by the specialized strain.

Santa Anna et al. (BR 0505299-8) teach a process for production of ethanol for the purpose of treatment of the solid residue resulting from the acid hydrolysis of sugar cane bagasse. According to this process a hydrolysate of the hemicellulosic fraction of sugar cane bagasse, rich in xylose, was obtained by means of mild hydrolysis with dilute sulphuric acid, and was fermented using a strain of the yeast *Pichia stipitis* suitably acclimatized to the main substrate of the hydrolysate (xylose). The solid residue resulting from acid hydrolysis (cellulignin) was treated in a special reactor for removal of lignin, by a series of alkaline washings to make the cellulosic fibres suitable for receiving an enzymatic charge.

Santa Anna et al. (BR 0605017-4) describe a process for obtaining ethanol from lignocellulosic materials enzymatically, according to which the hemicellulosic fraction is submitted to mild hydrolysis with sulphuric acid, and the solid material resulting from this hydrolysis is submitted to the process of saccharification (enzymatic hydrolysis) simultaneously with rapid alcoholic fermentation, in conditions permitting a significant increase in conversion to ethanol in much shorter times, using high concentrations of solids (15% to 25%).

Chung and Day (WO 2008/095098) present a process for obtaining sugars from lignocellulosic biomass in which the material is submitted to a hot alkaline pretreatment with a mixture of calcium hydroxide and water at a temperature from 80°C to 140°C for about 30 min to 3 hours. After the treatment, the bagasse is pressed; the liquid contains mainly soluble components of lignin, besides lime (which can be recovered) and the fibrous solid material is submitted to hydrolysis by cellulase enzymes. According to the authors, this treatment changes the lignocellulosic structure so that it can be quickly dissolved by cellulase, even using high solids contents (10% to 30%), without affecting the enzymatic activity. Commercial enzymes were used, for example, Spezyme CP (Genecor International Co.) and Novo I88 (Novozyme).

The aim of the process of the present invention is to offer an enzymatic preparation containing enzymes prepared *in situ* (or dedicated) of low cost, for economically scaling up biochemical technology for application in processes for obtaining ethanol from lignocellulosic materials. The preparation obtained can be applied to the solid produced after hydrolysis of the hemicellulosic fraction as well as fermentation processes that use simultaneous saccharification (SSF), as mentioned above.

### SUMMARY OF THE INVENTION

The process of the present invention is based on the microbial production of enzymes from growth of a *Penicillium funiculosum* fungus in a suitable culture medium with cellulosic substrate.

The invention relates to the production of cellulase enzymes by fermentation using the fungus *Penicillium funiculosum,* in which lignocellulosic residues are used, *in natura* or pretreated.

) The invention provides a process for producing an enzymatic concentrate, which enzymatic concentrate comprises enzymes for the hydrolysis of cellulose or hemicellulose from a lignocellulosic residue into fermentable sugars, wherein said process comprises:
- contacting a Penicillium funiculosum fungus with the lignocellulosic residue in a suitable culture medium for a period of four to seven days, and thereby producing said enzymes;
- concentrating said culture medium containing said enzymes using membranes or an evaporative process, thereby obtaining concentrate of said enzymes;
- adding a biosurfactant to the concentrate for improving the accessibility of said enzymes; and
- using the enzymatic concentrate for the hydrolysis of cellulose or hemicellulose from the lignocellulosic residue into fermentable sugars.

### DETAILED DESCRIPTION OF THE INVENTION

The process of the present invention comprises in particular the fermentative treatment of lignocellulosic ) and agricultural-forestry substrates by

means of a specially adapted P. funiculosum fungus, for the purpose of obtaining an enzymatic preparation capable of hydrolysing cellulose and hemicellulose for the production of alcohol.

The invention is based on the production of cellulase enzymes by fermentation using the fungus *Penicillium funiculosum* ATCC 11797, it being possible to use lignocellulosic substrates, *in natura* or pretreated.

The next stage of the process comprises concentration of the enzymes produced in fermentation, using membrane systems - such as a combined system of microfiltration and ultrafiltration, or by evaporation - for example the rotary evaporator. Finally, an additive is added to this concentrate of enzymes, in order to improve the activity of the enzymes in breaking the cellulose. This additive comprises a biosurfactant of the glycolipid type that promotes an increase in accessibility of the enzymes to cellulose and hemicellulose.

The enzymatic concentration is then used for the hydrolysis of the lignocellulosic residue into fermentable sugars.

For better understanding of the invention, the process can be described briefly as follows:
The P. funiculosum fungus is brought in contact with the lingocellulosic substrate in a suitable culture medium for a period of four to seven days, so that the enzymes are produced. During this time, the microorganism releases high concentrations of proteins with catalytic properties into the culture medium, i.e. cellulase and hemicellulase enzymes, such as xylanases, endoglucanases, exoglucanases and β-glucosidases, with the aim of breaking the cellulosic and hemicellulosic fractions present in the substrate. After this period, the culture medium containing the enzymes is submitted to a concentration process using membranes or evaporative processes, followed by application of additives.

The enzymatic preparation (extract) may be produced with residual materials from conventional production of ethanol, for example sugar cane bagasse and straw, with low production costs, so that it can be produced *in situ,* with the result that scaling-up of second-generation ethanol production becomes feasible.

Agricultural and forestry material, for example chips and residues from the pulp and paper industry, can also be used as the source of lignocellulose.

The examples given below are for purposes of illustration only, and do not represent any kind of limitation of the invention.

### EXAMPLE 1

This example aims to demonstrate the potential for use of the process now proposed for obtaining the enzymatic preparation.

Using the process of the invention as already described in detail, it was possible to produce an extract with high concentrations of cellulase enzymes, expressed in enzymatic activities (IU/L) and protein concentrations (mg/L). Table 1 given below presents the results of measurement of enzymatic activity of the extracts at the end of fermentation and after concentration.

| TABLE 1 | | | | |
|---|---|---|---|---|
| EXTRACT | FPase (U/L) | Endoglucanase (IU/L) | β-Glucosidase (IU/L) | Protein (mg/L) |
| Fermented | 125.161 | 2098.922 | 1016.784 | 90.999 |
| Concentrated | 5447.724 | 69276.758 | 40679.012 | 2781.543 |

These concentrated enzymatic extracts were tested in the process of hydrolysis (saccharification) of cellulose and hemicellulose contained in the pretreated sugar cane bagasse, i.e. submitted to mild hydrolysis and washing with heating for removal of lignin, and the results are shown in Table 2.

The measurement of sugars (glucose, xylose and cellobiose) was assessed by the methods of liquid chromatography (HPLC) and spectrophotometric measurement of total reducing sugars (TRS). This table also shows, for comparison, results obtained with a commercial preparation (GENENCOR^{®}), standardized to the same enzyme concentration per gram of cellulose.

| TABLE 2 | | | | |
|---|---|---|---|---|
| Enzymatic preparation | Glucose (g/L) | Xylose (g/L) | Cellobiose (g/L) | TRS (g/L) |
| GENENCOR® | 36.332 | 0.000 | 2.110 | 45.954 |
| Concentrated extract | 28.305 | 4.200 | 0.000 | 29.734 |

Table 2 shows the amounts of glucose obtained from the hydrolysis of cellulose. It can also be seen that the concentrated extract also contains xylanase enzymes that can convert hemicellulose to xylose, which it will also be possible to utilize for conversion to ethanol. This confirms that the enzymatic extract produced can be applied in processes of simultaneous saccharification and fermentation (SSF) and simultaneous saccharification and co-fermentation (SSCF).

### EXAMPLE 2

The purpose of this example is to demonstrate the applicability of the enzymatic preparation in the hydrolysis of lignocellulosic residue pretreated by mild hydrolysis and washing with heating to remove the lignin, obtained from the conventional production of ethanol from sugar cane.

The enzymatic preparation was applied in concentrations between 5 and 30 FPU/g, at temperatures between 30°C and 60°C, for 6 to 48 hours of saccharification.

The concentration of glucose released after the treatment varied from 10 to 40 g/L).

The results achieved enable us to assert that this is a considerable advantage of the extract produced by the process of the invention, by *P. funiculosum,* in relation to the commercial preparation assessed, as it indicates that the enzyme pool produced can be applied in processes of simultaneous saccharification and co-fermentation, which in terms of equipment is a more integrated and less complex form of processing.

Another advantage of the extract of *P. funiculosum* is the high β-glucosidase activity, which enables all of the disaccharide cellobiose produced to be converted to glucose (fermentable sugar), whereas with the commercial product there is still a residual amount of cellobiose, a disaccharide that cannot be utilized for obtaining ethanol in the fermentation process.

Another advantage observed is the synergistic action of the enzymes of this preparation, in the hydrolysis of cellulose and hemicellulose, breaking these polysaccharides to sugar monomers, without producing unfermentable residual intermediate sugars, which are not utilized for the production of ethanol.

Furthermore, it was produced from agroindustrial or agricultural-forestry residues that are abundant in Brazil, and applied *in situ* in the hydrolysis of these same residues, including residues with different compositions, achieving productivity similar to or greater than that resulting from commercial products.

Another great advantage of this invention relates to the high cost of the cellulase enzymes offered in the market, a fact that up to now has prevented the scaling-up of the biochemical technology for conversion of cellulosic materials to ethanol. This invention makes possible the production of these enzymes "in *situ"* or dedicated, greatly reducing their cost of production.

## Claims

1. A process for producing an enzymatic concentrate, which enzymatic concentrate comprises enzymes for the hydrolysis of cellulose or hemicellulose from a lignocellulosic residue into fermentable sugars, wherein said process comprises:
- contacting a Penicillium funiculosum fungus with the lignocellulosic residue in a suitable culture medium for a period of four to seven days, and thereby producing said enzymes;
- concentrating said culture medium containing said enzymes using membranes or an evaporative process, thereby obtaining a concentrate of said enzymes;
- adding a biosurfactant to the concentrate for improving the accessibility of said enzymes; and
- using the enzymatic concentrate for the hydrolysis of cellulose or hemicellulose from the lignocellulosic residue into fermentable sugars.

2. The process according to claim 1 wherein said process comprises simultaneous saccharification and fermentation (SSF), or simultaneous saccharification and co-fermentation (SSCF).

3. The process according to claim 1 or claim 2 wherein said process further comprises hydro lysing the lignocellulosic residue, wherein the residue comprises sugar cane bagasse *in natura* or pretreated.

4. The process according to claim 3 wherein the residue is pretreated by submitting it to mild hydrolysis and washing with heating to remove lignin.

5. The process according to claim 3 or claim 4 wherein the residue comprises agricultural-forestry material.

6. The process according to any one of claims 1 to 5 wherein the concentrate is used in the hydrolysis of cellulose and/or hemicelluloses to produce sugar monomers, without producing unfermentable residual intermediate sugars.

7. The process according to any one of claims 1 to 6 wherein the concentrate is *produced in situ,* with residual materials from the conventional production of ethanol from sugar cane.

8. The process according to any one of claims 1 to 7 wherein the biosurfactant is of the glycolipid type.

9. The process according to any one of claims 1 to 8 wherein the concentrate is produced *in situ* with residual materials which are *in natura* and/or pretreated and which comprise residual materials from the conventional production of ethanol from sugar cane, such as bagasse and straw, and/or residual materials from the pulp and paper industry, such as chips.

## Patentansprüche

1. Verfahren zur Herstellung eines enzymatischen Konzentrates, wobei das enzymatische Konzentrat Enzyme für die Hydrolyse von Cellulose oder Hemicellulose aus einem lignozellulosehaltigen Rückstand in fermentierbare Zucker umfasst, wobei das Verfahren umfasst:
- Inkontaktbringen eines Penicillium-funiculosum-Pilzes mit dem lignozellulosehaltigen Rückstand in einem geeigneten Kulturmedium für einen Zeitraum von vier bis sieben Tagen und dadurch Produzieren der Enzyme;
- Konzentrieren des Kulturmediums, das die Enzyme enthält, unter Verwendung von Membranen oder einem Verdampfungsverfahren, wodurch ein Konzentrat der Enzyme erhalten wird;
- Zugeben eines Biosurfactants zu dem Konzentrat zur Verbesserung der Akzessibilität der Enzyme und
- Verwenden des enzymatischen Konzentrates für die Hydrolyse von Cellulose oder Hemicellulose aus dem lignozellulosehaltigen Rückstand in fermentierbare Zucker.

2. Verfahren gemäß Anspruch 1, wobei das Verfahren gleichzeitige Verzuckerung und Fermentation (simultaneous saccharification and fermentation (SSF)) oder gleichzeitige Verzuckerung und Cofermentation (simultaneous saccharification and co-fermentation (SSCF)) umfasst.

3. Verfahren gemäß Anspruch 1 oder Anspruch 2, wobei das Verfahren außerdem Hydrolysieren des lignozellulosehaltigen Rückstandes umfasst, wobei der Rückstand Zuckerrohr-Bagasse in natura oder vorbehandelt umfasst.

4. Verfahren gemäß Anspruch 3, wobei der Rückstand vorbehandelt wird, indem er einer milden Hydrolyse und einem Waschen unter Erwärmen unterzogen wird, um Lignin zu entfernen.

5. Verfahren gemäß Anspruch 3 oder Anspruch 4, wobei der Rückstand ein landwirtschaftlich-forstwirtschaftliches Material umfasst.

6. Verfahren gemäß einem der Ansprüche 1 bis 5, wobei das Konzentrat bei der Hydrolyse von Cellulose und/oder Hemicellulose verwendet wird, um Zuckermonomere herzustellen, ohne nichtfermentierbare restliche Intermediatzucker herzustellen.

7. Verfahren gemäß einem der Ansprüche 1 bis 6, wobei das Konzentrat mit Rückstandsmaterialien aus der herkömmlichen Produktion von Ethanol aus Zuckerrohr in situ produziert wird.

8. Verfahren gemäß einem der Ansprüche 1 bis 7, wobei das Biosurfactant vom Glycolipidtyp ist.

9. Verfahren gemäß einem der Ansprüche 1 bis 8, wobei das Konzentrat in situ mit Rückstandsmaterialien produziert wird, die in natura und/oder vorbehandelt sind und die Rückstandsmaterialien aus der herkömmlichen Produktion von Ethanol aus Zuckerrohr, zum Beispiel Bagasse und Stroh, und/oder Rückstandsmaterialien aus der Pulpe- und Papierindustrie, zum Beispiel Späne, umfassen.

## Revendications

1. Procédé de production d'un concentré enzymatique, lequel concentré enzymatique comprend des enzymes servant à hydrolyser de la cellulose ou de l'hémicellulose issue d'un résidu lignocellulosique en sucres fermentescibles, lequel procédé comporte les étapes suivantes :
- mettre du champignon *Penicillium funiculosum* en contact avec le résidu lignocellulosique, dans un milieu de culture approprié, durant quatre à sept jours, et produire ainsi lesdites enzymes ;
- concentrer ledit milieu de culture contenant lesdites enzymes en se servant de membranes ou en appliquant un procédé d'évaporation, et obtenir ainsi un concentré desdites enzymes ;
- ajouter au concentré un biotensioactif, pour améliorer l'accessibilité de ces enzymes ;
- et utiliser ce concentré enzymatique pour l'hydrolyse de la cellulose ou de l'hémicellulose issue du résidu lignocellulosique en sucres fermentescibles.

2. Procédé conforme à la revendication 1, lequel procédé comprend saccharification et fermentation simultanées (SFS), ou saccharification et co-fermentation simultanées (SCFS).

3. Procédé conforme à la revendication 1 ou 2, lequel procédé comprend en outre l'hydrolyse du résidu lignocellulosique, lequel résidu comprend de la bagasse de canne à sucre, au naturel ou prétraitée.

4. Procédé conforme à la revendication 3, pour lequel on pré-traite le résidu en lui faisant subir une hydrolyse modérée et un lavage à chaud pour éliminer la lignine.

5. Procédé conforme à la revendication 3 ou 4, dans lequel le résidu comprend un matériau agro-sylvicole.

6. Procédé conforme à l'une des revendications 1 à 5, dans lequel on utilise le concentré dans l'hydrolyse de la cellulose et/ou des hémicelluloses pour produire des monomères de sucre sans produire des sucres intermédiaires résiduels non-fermentescibles.

7. Procédé conforme à l'une des revendications 1 à 6, dans lequel le concentré est produit *in situ* avec des résidus provenant de la production classique d'éthanol à partir de sucre de canne.

8. Procédé conforme à l'une des revendications 1 à 7, dans lequel le biotensioactif est de type glycolipide.

9. Procédé conforme à l'une des revendications 1 à 8, dans lequel le concentré est produit *in situ* avec des résidus qui sont au naturel et/ou prétraités et qui comprennent des résidus provenant de la production classique d'éthanol à partir de sucre de canne, comme de la bagasse et de la paille, et/ou des résidus provenant de l'industrie de la pâte à papier et du papier, comme des copeaux.
